(19) Europäisches Patentamt
European Patent Office
Office européen des brevets

(11) Veröffentlichungsnummer : **0 357 978 B1**

(12) # EUROPÄISCHE PATENTSCHRIFT

(45) Veröffentlichungstag der Patentschrift :
**13.05.92 Patentblatt 92/20**

(51) Int. Cl.$^5$ : **A61K 37/26,** C07K 7/40

(21) Anmeldenummer : **89114617.7**

(22) Anmeldetag : **08.08.89**

(54) **Pharmazeutische Zubereitung zur Behandlung des Diabetes mellitus.**

Die Akte enthält technische Angaben, die nach dem Eingang der Anmeldung eingereicht wurden und die nicht in dieserPatentschrift enthalten sind.

(30) Priorität : **13.08.88 DE 3827533**

(43) Veröffentlichungstag der Anmeldung :
**14.03.90 Patentblatt 90/11**

(45) Bekanntmachung des Hinweises auf die Patenterteilung :
**13.05.92 Patentblatt 92/20**

(84) Benannte Vertragsstaaten :
**AT BE CH DE ES FR GB GR IT LI LU NL SE**

(56) Entgegenhaltungen :
**EP-A- 0 137 361**
**EP-A- 0 140 084**

(73) Patentinhaber : **HOECHST AKTIENGESELLSCHAFT**
**Postfach 80 03 20**
**W-6230 Frankfurt am Main 80 (DE)**

(72) Erfinder : **Dörschug, Michael, Dr.**
**Sonnenleite 20**
**W-4630 Bochum 7 (DE)**

**EP 0 357 978 B1**

Anmerkung : Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Therapie des Diabetes mellitus erfolgt heute überwiegend durch parenterale Verabreichung von Zubereitungen des blutzuckersenkenden Hormons Insulin. Ziel dieser Therapie ist es, den menschlichen Organismus dem Zustand seines natürlichen hormonellen Gleichgewichts möglichst weitgehend anzunähern, d.h. den Patienten möglichst optimal "einzustellen", denn bei nicht optimaler "Einstellung" ist neben unmittelbaren Folgen wie Hyper- oder Hypoglykämien insbesondere mit diabetischen Spätkomplikationen zu rechnen, zu denen u.a. die Retinopathie, Neuropathie, Nephropathie, Mikro- und Makroangiopathie zu zählen sind.

Wegen der Verschiedenheit der menschlichen Individuen - und damit natürlich auch der Diabetes mellitus-Patienten - ist es erforderlich, für die möglichst optimale "Einstellung" der Patienten eine Vielzahl von Insulin-Zubereitungen mit verschiedenen Wirkungscharakteristiken verfügbar zu haben.

Wegen der speziellen Natur des Insulins und dessen Metabolismus ist die Wirkungsdauer einer einfachen Insulinlösung nur sehr kurz, so daß zur anhaltenden Blutzuckerkontrolle beim Diabetiker entweder mehrfache tägliche Injektionen oder eine Dauerinfusion mit Dosiergeräten erforderlich sind oder eine verzögert wirksame Insulinzubereitung appkliziert werden muß.

bei unmodifiziertem Insulin in gelöster Form (bei saurem pH-Wert) kann ein Verzögerungsprofil durch die Gegenwart hoher Mengen von Zinkionen - z.B. 0,4-1 mg/IE (= internationale Einheit) Insulin - erreicht werden; vergleiche J Pharmakol. 55 (1935), S. 206. Derartig hohe Zinkmengen verursachen bei der Applikation jedoch Schmerzen, so daß solche Insulinlösungen mit so hohen Zinkgehalten in der Diabetes-Therapie nicht zur Anwendung kamen.

Von erheblicher therapeutischer Bedeutung als Verzögerungsprinzipien sind solche Zustandsformen des Insulins, die am Ort der Injektion schwerlöslich sind. Dazu zählen beispielsweise Zink-Insulinkristalle oder Protamin-Insulin-Kristalle, die während ihrer langsamen Widerauflösung über einen gewissen Zeitraum Insulin freisetzen. Die gebräuchlichen Verzögerungssuspensionen von Zink-Insulinkristallen bzw. Zink- Protamin-Insulin müssen vor der Applikation homogen durchmischt werden.

Ein vorteilhaftes weiteres Verzögerungsprinzip stellen die basisch modifizierten Insulinderivate gemäß EP-B-0132 770 dar. Es handelt sich um speziell in der Position B31 der Insulin-B-kette basisch modifizierte Derivate mit einem isoelektrischen Punkt zwischen 5,8 und 8,5. Die entsprechenden Arzneimittel enthalten als Wirkstoff wenigstens ein - in Position B31 basisch modifiziertes - Insulinderivat der nachstehenden Formel I und/oder wenigstens eines von dessen physiologisch verträglichen Salzen; Formel I ist

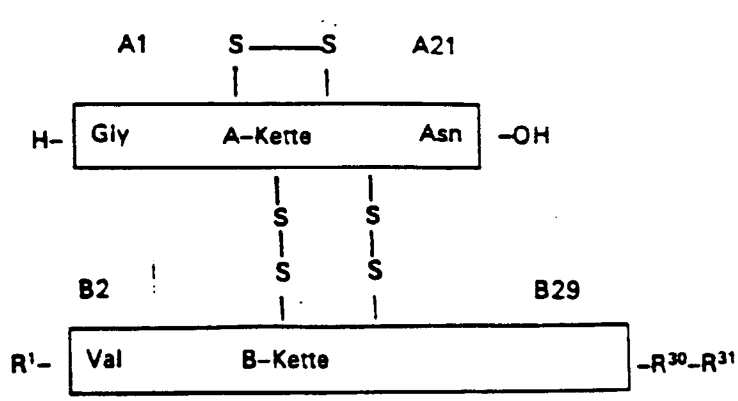

(I)

in welcher $R^1$ H oder H-Phe bedeutet,
$R^{30}$ für den Rest einer neutralen, genetisch kodierbaren L-Aminosäure steht und
$R^{31}$ für eine physiologisch unbedenkliche organische Gruppe basischen Charakters mit bis zu 50 C-Atomen steht, an deren Aufbau 0 bis 3 $\alpha$-Aminosäuren beteiligt sind und deren gegebenenfalls vorhandene endständige Carboxylfunktion frei, als Esterfunktion, als Amidfunktion, als Lacton oder zu $CH_2OH$ reduziert vorliegen kann.

Die Verzögerungs- oder Depot-Wirkung dieser basisch modifizierten Insulinderivate geht auf eine inhärentes, proteinchemisch bedingtes, physikalisches Prinzip, nämlich die Schwerlöslichkeit des Insulinderivats an dessen isoelektrischem Punkt, zurück. Seine Wiederauflösung unter physiologischen Bedingungen soll nach der vorerwähnten EP-B-0 132 770 Abspaltung der zusätzlichen basischen Gruppen erreicht, die je nach Derivat durch tryptische oder Trypsin-ähnliche und/oder Carboxypeptidase B oder der Carboxypeptidase B ähnliche und/oder Esterase-Aktivität zustande kommt. Die jeweils abgespaltenen Gruppen sind entweder rein physiologische Metaboliten oder aber leicht metabolisierbare, physiologisch unbedenkliche Substanzen.

Die pharmazeutischen Zubereitungen dieser basisch modifizierten Insulinderivate können zinkfrei sein

oder auch bis zu 100 µg Zink/100 IE (= 1 µg Zink/IE) enthalten.

Die in B31-Position der Insulin-B-Kette basisch modifizierten Insulinderivate und deren physiologisch verträglich Salze können gemäß EP-B-0132769 auch u.a. mit unmodifiziertem Insulin und/oder dessen physiologisch unbedenklichen Salzen gemischt werden; die entsprechenden pharmazeutischen Zubereitungen weisen ein Wirkungsprofil auf, welches sich aus den Wirkungsprofilen der einzelenen Wirkstoffe zusammensetzt.

Auch diese Misch-Zubereitungen können zinkfrei sein oder einen Zinkgehalt bis zu 1 µg/IE enthalten.

Das vorerwähnte Depotprinzip infolge basischer Modifikation des Insulins wurde noch weiter genutzt durch die Bereitstellung und entsprechende Verwendung anderer - hauptsächlich innerhalb der A- und B-Ketten - basisch modifizierter Insulinderivate; vergleiche EP-A-0194864 und EP-A-0254516. Die pharmazeutischen Zubereitungen mit diesen speziellen basisch modifizierten Insulinderivaten enthalten vorzugsweise Zinkionen in einer Menge zwischen 2 µg und etwa 2 mg/ml, insbesondere zwischen 5 µg und 200 µg/ml. Durch Mischung einer zinkfreien Wirkstofflösung oder -Suspension mit einer separaten Zinksalzlösung unmittelbar vor der Applikation kann gegebenenfalls der Patient selbst einen jeweils unterschiedlichen Zinkgehalt der Applikationsform - und damit ein den Umständen besser angepaßtes - Wirkungsprofil einstellen.

Trotz der erheblichen Zahl bekannter Insulinformen mit schneller und auch mit verzögerter Wirkung sowie auch mit "gemischten" Wirkungsprofilen besteht wegen der individuellen Verschiedenheit der Lebewesen ein Bedarf an weiteren Insulin-Applikationsformen mit weiteren speziellen Wirkungsprofilen.

In dem Bestreben, weitere solche Insulin-Applikationsformen mit speziellen Wirkungsprofilen bereitzustellen, wurde nun gefunden, daß dieses Ziel durch eine Erhöhung des Zinkgehalts der in den beiden vorerwähnten EP-Patentschriften 0132770 und 0132769 beschriebenen pharmazeutischen Zubereitungen erreicht wird.

Erfindungsgegenstand ist daher eine pharmazeutische Zubereitung, enthaltend mindestens ein basisch modifiziertes Insulinderivat mit einem isoelektrischen Punkt zwischen 5,8 und 8,5 der vorher bereits erwähnten Formel I

und/oder mindestens eines von dessen physiologisch verträglichen Salzen

in einem pharmazeutisch unbedenklichen Träger mit einem Gehalt an Zinkionen;

die Zubereitung ist dadurch gekennzeichnet, daß der Zinkionengehalt in dem Bereich von oberhalb 1 µg bis etwa 200 µg Zink/IE, vorzugsweise von etwa 1 bis 50 µg Zink/IE, liegt.

Es ist überraschend, daß der relativ hohe Zinkgehalt hier zu keinen Komplikationen bei den Patienten führt. Durch den Zinkgehalt innerhalb der angegebenen Grenzen und das in derartigen Zubereitungen obligatorische Isotonikum läßt sich das Wirkprofil in einem breiten Rahmen steuern. Besonders vorteilhaft ist hier die Kombination von hohen Zinkgehalten und bestimmten Isotonika, die das Insulinderivat trotz des hohen Zinkgehalts in schwach saurem Milieu in Lösung halten. Ein schwach saurer pH-Bereich ist vorteilhaft wegen der in diesem Bereich bekannten Langzeitstabilität der Insulinderivate, bei denen es unter stärker sauren Bedingungen zu Derivatbildungen (insbesondere zur Desamidierung $Asn^{A21}$) kommt. Die hier eingesetzten Insulinderivate sind außerdem in Gegenwart der hohen Zinkkonzentrationen biologisch voll wirksam.

Die für die erfindungsgemäße Zubereitung in Frage kommenden basisch modifizierten Insulinderivate der Formel I sind die in der Position B31 basisch modifizierten Insulinderivate wie sie in den eingangs erwähnten Druckschriften EP-B0132770 und EP-B0132769 für die dortigen Arzneimittel ohne oder mit einem geringeren Zinkgehalt beschrieben sind. Es handelt sich also um Verbindungen der Formel I, in welcher

$R^1$H oder H-Phe bedeutet,

$R^{30}$ für den Rest einer neutralen, genetisch kodierbaren L-Aminosäure steht und

$R^{31}$ für eine physiologisch unbedenkliche organische Gruppe basischen Charakters mit bis zu 50 C-Atomen steht, an deren Aufbau 0 bis 3 α-Aminosäuren beteiligt sind und deren gegebenenfalls vorhandene entständige Carboxylfunktion frei, als Esterfunktion, als Amidfunktion, als Lacton oder zu $CH_2OH$ reduziert vorliegen kann.

$R^1$ ist bevorzugt H-Phe.

Neutrale, genetisch kodierbare L-Aminosäuren - für R30 - sind Gly, Ala, Ser, Thr, Val, Leu, Ile, Asn, Gln, Cys, Met, Tyr, Phe und Pro; bevorzugt sind Ala, Ser und Thr, insbesondere Thr.

$R^{31}$ ist eine physiologisch unbedenkliche organische Gruppe basischen Charakters mit bis zu 50 C-Atomen, an deren Aufbau 0 - 3α Aminosäuren beteiligt sind. Wenn am Aufbau von $R^{31}$ keine α-Aminosäuren beteiligt sind, kommen für diesen Rest z.B. folgende basischen Gruppen in Frage: Amino- ($C_2$ bis $C_6$)-alkoxy, ($C_1$ bis $C_4$)-Alkylamino-($C_2$ bis $C_6$)-alkoxy, Di-($C_1$ bis $C_4$)-alkylamino-($C_2$ bis $C_6$)-alkoxy, Tri-($C_1$ bis $C_4$)ammonio-($C_2$ bis $C_6$)-alkoxy, Amino ($C_2$ bis $C_6$)-alkylamino, [($C_1$ bis $C_4$)-Alkylamino]-($C_2$ bis $C_6$)alkylamino, [Di-($C_1$-$C_4$)-alkylamino]-($C_2$-$C_6$)-alkylamino oder [Tri-($C_1$ bis $C_4$)-alkylamino]-($C_2$ bis $C_6$)-alkylamino, insbesondere $-O-[CH_2]_p NR_2$, $-O-[CH_2]_p-N^+R_3$, $-NH-[CH_2]_p-NR_2$ oder $-NH-[CH_2]_p-N^+R_3$, worin p = 2 bis 6 ist und R gleich oder verschieden ist und für Wasserstoff oder ($C_1$ bis $C_4$)-Alkyl steht.

Wenn am Aufbau von $R^{31}$ bis zu 3 α-Aminosäuren beteiligt sind, sind dies in erster Linie neutrale oder basische natürlich vorkommende L-Aminosäuren und/oder die diesen entsprechenden D-Aminosäuren. Neutrale natürlich vorkommende Aminosäuren sind insbesondere Gly, Ala, Ser, Thr, Val, Leu, Ile, Asn, Gln, Cys, Met,

Tyr, Phe, Pro und Hyp. Basische natürlich vorkommende Aminosäuren sind insbesondere Arg, Lys, Hyl, Orn, Cit und His. Falls nur neutrale α-Aminosäuren beteiligt sind, kann deren endständige Carboxylfunktion - damit $R^{31}$ basischen Charakter hat - nicht frei sein; die Caboxylfunktion muß vielmehr in diesem Fall mit einer basischen Gruppe verestert oder amidiert sein, wobei als solche basischen Gruppen etwa die vorher - für den Fall, daß am Aufbau von $R^{31}$ keine α-Aminosäuren beteiligt sind - erwähnten basischen Gruppen in Frage kommen. Natürlich können diese basischen Ester- oder Amidgruppen auch die Carboxylfunktion von basischen α-Aminosäuren blockieren. Für die Blockierung der Carboxylfunktion der basischen -Aminosäuren können - falls die Blockierung gewünscht ist - auch neutrale Ester- oder Amidgruppen wie z.B. ($C_1$ bis $C_6$)-alkoxy, ($C_3$ bis $C_6$)-Cykloalkyloxy, $NH_2$, ($C_1$ bis $C_6$)-alkylamino oder Di-($C_1$ bis $C_6$)-alkylamino in Frage kommen.

Als Lacton kann die endständige Carboxylfunktion natürlich nur vorliegen, wenn die endständige Aminosäure eine Hydroxyaminosäure ist.

Außerdem kann die endständige Carboxylfunktion auch zu $CH_2OH$ reduziert sein.

Bevorzugt besteht $R^{31}$ aus 1,2 oder 3 der vorerwähnten basischen natürlich vorkommenden Aminosäuren; besonders bevorzugt ist $R^{31}$ = Arg-OH oder Arg-Arg-OH.

Die (A1 bis A21)- und die (B2 bis B29)-Sequenzen der Insulinderivate der Formel I sind vorzugsweise die Sequenzen des Human-, Schweine- oder Rinder-Insulins, insbesondere die Sequenzen des Human-Insulins.

Konkrete basisch modifizierte Insulinderivate der Formel I sind z.B.:

Humaninsulin-$Arg^{B31}$-OH
Humaninsulin-$Arg^{B31}$-$Arg^{B32}$-OH
Des-$Phe^{B1}$-Schweineinsulin-$Arg^{B31}$-OH
Des-$Phe^{B1}$-Humaninsulin-$Arg^{B31}$-OH
Des-$Phe^{B1}$-Schweineinsulin-$Arg^{B31}$-$Arg^{B32}$-OH
Des-$Phe^{B1}$-Humaninsulin-$Arg^{B31}$-$Arg^{B32}$-OH
Schweineinsulin-$Arg^{B31}$-$OCH_3$
Humaninsulin-$Arg^{B31}$-$OCH_3$
Rinderinsulin-$Arg^{B31}$-$OCH_3$
Schweineinsulin-$Arg^{B31}$-$Arg^{B32}$-$OCH_3$
Humaninsulin-$Arg^{B31}$-$Arg^{B32}$-$OCH_3$
Des-$Thr^{B30}$-Humaninsulin-$Val^{B30}$-$Arg^{B31}$-OH
Des-$Thr^{B30}$-Humaninsulin-$Val^{B30}$-$Ala^{B31}$-$Arg^{B32}$-OH
Humaninsulin-$Lys^{B31}$-OH
Humaninsulin-D-$Arg^{B31}$-OH
Humaninsulin-D-$Arg^{B31}$-$Arg^{B32}$-OH
Humaninsulin-$Arg^{B31}$-D-$Arg^{B32}$-OH
Humaninsulin-$Lys^{B31}$-$Arg^{B32}$-OH
Humaninsulin-$Arg^{B31}$-$Lys^{B32}$-OH
Humaninsulin-$Argininol^{B31}$
Humaninsulin-$Val^{B31}$-$Arg^{B32}$-OH
Humaninsulin-$Val^{B31}$-$Arg^{B32}$-$Arg^{B33}$-OH
Humaninsulin-$Arg^{B31}$-$Argininol^{B32}$
Humaninsulin-$Lys^{B31}$-$Arg^{B32}$-$Arg^{B33}$-OH

Humaninsulin-$Arg^{B31}$-NH
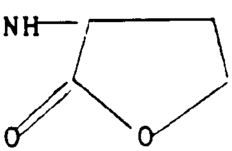

Humaninsulin-$Arg^{B31}$-$Arg^{B32}$-NH
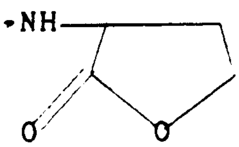

Humaninsulin-$Arg^{B31}$-$NH_2$
Humaninsulin-$Arg^{B31}$-$Arg^{B32}$-$NH_2$
Humaninsulin-$Orn^{B31}$-OH
Humaninsulin-$Leu^{B31}$-$Cit^{B32}$-OH

Humaninsulin-(B30)-$OCH_2CH_2$-$NH_2$
Humaninsulin-(B30)-NH-$CH_2CH_2$-$NH_2$
Humaninsulin-$Arg^{B31}$-O-$CH_2$-$CH_2$-$NH_2$
Humaninsulin-$Arg^{B31}$-$CH_2$-$CH_2$-$N(CH_3)_2$
Humaninsulin-(B30)-O-$CH_2$-$CH_2$-$N(CH_3)_3$
Humaninsulin-(B30)-NH-$CH_2$-$CH_2$-$N(CH_3)_3$
Humaninsulin-$Leu^{B31}$-O-$CH_2$-$CH_2$-$CH_2$-$N(C_2H_5)_3$
Humaninsulin-$Trp^{B31}$-$Trp^{B32}$-$Trp^{B33}$-$NH(CH_2)_6$-$N((CH_2)_3CH_3)_3$

Auch die physiologisch verträglichen Salze dieser basisch modifizierten Insulinderivate können verwendet werden.

Der Zinkionengehalt wird bewirkt Zinksalze wie z.B. $ZnCl_2$, $ZnSO_4$.

Ansonsten kann die erfindungsgemäße pharmazeutische Zubereitung aufgebaut sein wie dies auch für die Arzneimittel in den vorerwähnten EP-B0132770 und EP-B-0132769 beschrieben ist. Sie weist vorzugsweise einen pH-Wert zwischen etwa 2,5 und 8,5, insbesondere zwischen etwa 4,0 und 8,5 auf, enthält ein geeignetes Isotoniemittel, ein geeignetes Konservierungsmittel und gegebenenfalls einen geeigneten Puffer, alles natürlich in steriler wäßriger Lösung. Die Gesamtheit der Zubereitungsbestandteile außer den Wirkstoffen bildet den Zubereitungs-Träger.

Geeignete Isotoniemittel sind z.B. Glyzerin, Glukose, NaCl, Calcium- oder Magnesium-Verbindungen wie $CaCl_2$, $MgCl_2$.

Durch die Wahl des Isotoniemittels beeinflußt man die Löslichkeit des basisch modifizierten Insulinderivats bei den schwach sauren pH-Werten. Das Vorliegen eines gelösten Insulinderivates beinur schwach sauren pH-Wert ist wünschenswert, da es in diesem Bereich weniger stark zu Derivatbildung, insbesondere zur Bildung von Desamidierungsprodukten (z.B. $Asn^{21}$) kommt.

Geeignete Konservierungsmittel sind z.B. Phenol, m-Kresol, Benzylalkohol und/oder p-Hydroxybenzoesäureester.

Als Puffersubstanzen, insbesondere zur Einstellung eines pH-Werts zwischen etwa 4,0 und 8,5, können z.B. Natriumacetat, Natriumcitrat, Natriumphosphat verwendet werden. Ansonsten sind zur Einstellung des pH-Wertes auch physiologisch unbedenkliche verdünnte Säuren (typischerweise HCl) bzw. Laugen (typischerweise NaOH) geeignet.

Zwecks Variation des Wirkungsprofils der erfindungsgemäßen Zubereitung kann auch unmodifiziertes Insulin, vorzugsweise Rinder-, Schweine- oder Humaninsulin, insbesondere Humaninsulin, zugemischt werden.

Die nachfolgenden Beispiele sollen die Erfindung näher erläutern.

1. Wirkprofil einer InsulinArg$^{B31}$-OH Zubereitung am Hund in Abhängigkeit vom $Zn^{2+}$-Gehalt

Zusammensetzung/ml

| | |
|---|---|
| 40 IE | HumaninsulinArg$^{B31}$-OH |
| 18,82 mg | Glycerin |
| 10 mg | Benzylalkohol |
| pH | 4,0 |

Blutzucker in Prozent des Anfangswertes

| | Präparat nach | 1 H (=Stunde) | 2 H | 3 H | 5 H |
|---|---|---|---|---|---|
| Vgl. | 0 µg $Zn^{2+}$ | 60 | 50 | 72 | 110 |
| | 10 µg $Zn^{2+}$ | 51 | 51 | 72 | 98 |
| | Basal-H-Insulin Hoechst$^{(R)}$, d.i. eine NPH- (= neutral-Protamin nach Hagedorn)- mit ca. 10 µg $Zn^{2+}$ | 70 | 62 | 71 | 90 |
| erf.-gemäß | 80 µg $Zn^{2+}$ (= 2 µg/IE) | 60 | 51 | 52 | 70 |

2. Wirkprofil einer InsulinArg$^{B31}$-OH Zubereitung am Hund und am Kaninchen in Abhängigkeit vom $Zn^{2+}$-

Gehalt
Zusammensetzung/ml:

| | |
|---|---|
| 40 IE | HumaninsulinArg$^{B31}$-OH |
| 18,82 mg | Glycerin |
| 10 mg | Benzylalkohol |
| pH | 4,0 |

Blutzucker in Prozent des Anfangswertes

a) Hund

| | Präparat | 1 H | 2 H | 3 H | 5 H | 7 H |
|---|---|---|---|---|---|---|
| Vgl. | 40 µg $Zn^{2+}$ | 82 | 60 | 62 | 88 | 93 |
| | Depot-H-Insulin Hoechst(R) = 75 % NPH + 25 % Humaninsulin mit ca. 10 µg $Zn^{2+}$ | 63 | 51 | 61 | 100 | 101 |
| erf.-gemäß | 80 µg $Zn^{2+}$ | 69 | 52 | 51 | 70 | 93 |
| | 160 µg $Zn^{2+}$ | 95 | 68 | 60 | 70 | 82 |

Blutzucker in Prozent des Anfangswertes

b) Kaninchen

| | Präparat | 1 H | 2 H | 3 H | 5 H | 7 H |
|---|---|---|---|---|---|---|
| Vgl. | 40 µg $Zn^{2+}$ | 51 | 72 | 100 | 99 | 99 |
| | Depot-H-Insulin Hoechst(R) | 51 | 52 | 71 | 96 | 100 |
| erf.-gemäß | 80 µg $Zn^{2+}$ | 50 | 63 | 94 | 110 | 100 |
| | 160 µg $Zn^{2+}$ | 57 | 65 | 94 | 102 | 100 |

3. Wirkprofil einer InsulinArg$^{B31}$-OH Zubereitung am Hund in Abhängigkeit vom Isotonikum
Zusammensetzung/ml

| | |
|---|---|
| 40 IE | HumaninsulinArg$^{B31}$-OH |
| 10 mg | Benzylalkohol |
| 80 µm | $Zn^{2+}$ |
| pH | 4,5 |

| Präparat | Blutzucker in Prozent des Anfangswertes 1 H | 2 H | 3 H | 5 H | 7 H |
|---|---|---|---|---|---|
| mit Isotonikum: | | | | | |
| NaCl | 70 | 61 | 61 | 103 | 105 |
| $CaCl_2$ | 71 | 62 | 65 | 99 | 100 |
| Glycerin | 70 | 63 | 55 | 80 | 98 |
| Glucose | 70 | 61 | 58 | 91 | 108 |
| Natriumcitrat | 61 | 63 | 80 | 119 | 118 |
| Vgl.: Basal-H-Insulin HOECHST(R) | 71 | 55 | 63 | 85 | 95 |

4. Wirkprofil einer InsulinArg$^{B31-B32}$-OH Zubereitung am Kaninchen und am Hund in Abhängigkeit von $Zn^{2+}$-Gehalt

Zusammensetzung/ml

| | |
|---|---|
| 40 IE | HumaninsulinArg$^{B31-B32-OH}$ |
| 10 mg | Benzylalkohol |
| 18,82 mg | Glycerin |
| pH | 4,0 |

| a) Hund | | Blutzucker in Prozent des Anfangswertes 1 H | 2 H | 3 H | 5 H | 7 H |
|---|---|---|---|---|---|---|
| Vgl. | ohne $Zn^{2+}$ | 59 | 60 | 79 | 105 | 110 |
| | 5 µg $Zn^{2+}$ | 68 | 59 | 77 | 100 | 107 |
| | Basal-H-Insulin Hoechst(R) | 71 | 49 | 59 | 83 | 100 |
| erf.-gemäß | 80 µg $Zn^{2+}$ | 77 | 52 | 64 | 85 | 98 |

| b) Kaninchen | | 1 H | 2 H | 3 H | 5 H | 7 H |
|---|---|---|---|---|---|---|
| Vgl. | ohne $Zn^{2+}$ | 43 | 58 | 72 | 92 | 94 |
| | 5 µg $Zn^{2+}$ | 75 | 64 | 80 | 99 | 105 |
| | Basal-H-Insulin Hoechst(R) | 56 | 56 | 71 | 96 | 99 |
| erf.-gemäß | 80 µg $Zn^{2+}$ | 63 | 64 | 88 | 95 | 105 |

Die 7 H den Beispielen besitzen wegen der Überlagerung mehrerer verschiedener Einflüsse kaum mehr eine zuverlässige Aussagekraft.

## Patentansprüche

### Patentansprüche für folgende Vertragsstaaten : AT, BE, CH, DE, FR, GB, IT, LI, LU, NL, SE

1. Pharmazeutische Zubereitung, enthaltend mindestens ein basisch mofifiziertes Insulinderivat mit einem isoelektrischen Punkt zwischen 5,8 und 8,5 der Formel I

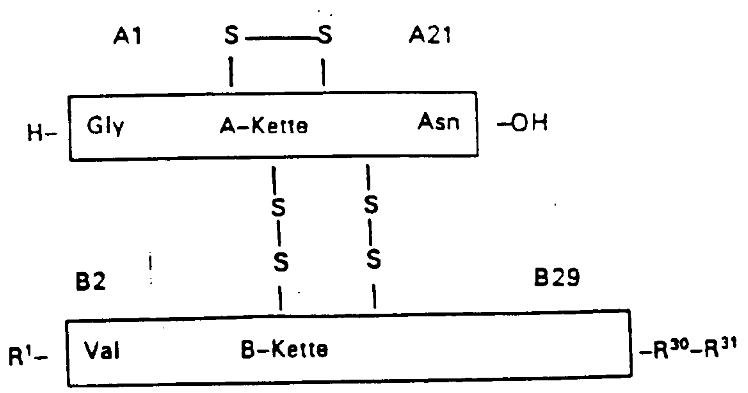

(I)

in welcher $R^1$ H oder H-Phe bedeutet,
$R^{30}$ für den Rest einer neutralen, genetisch kodierbaren L-Aminosäure steht und
$R^{31}$ für eine physiologisch unbedenkliche organische Gruppe basischen Charakters mit bis zu 50 C-Atomen steht, an deren Aufbau 0 bis 3 $\alpha$-Aminosäuren beteiligt sind und deren gegebenenfalls vorhandene endständige Carboxylfunktion frei, als Esterfunktion, als Amidfunktion, als Lacton oder $CH_2OH$ reduziert vorliegen kann,
und/oder mindestens eines von dessen physiologisch verträglichen Salzen in einem pharmazeutisch unbedenklichen Träger
mit einem Gehalt an Zinkionen,
dadurch gekennzeichnet, daß der Zinkionengehalt in einem Bereich von oberhalb 1 µg bis etwa 200 µg Zink/IE, vorzugsweise von etwa 1 bis 50 µg/IE, liegt.

2. Pharmazeutische Zubereitung nach Anspruch 1, dadurch gekennzeichnet, daß in Formel I $R^1$ H-Phe bedeutet.

3. Pharmazeutische Zubereitung nach Anspruch 1 oder 2, dadurch gekennzeichnet, daß in Formel I $R^{30}$ Ala, Ser oder Thr, vorzugsweise Thr bedeutet.

4. Pharmazeutische Zubereitung nach einem oder mehreren der Ansprüche 1 bis 3, dadurch gekennzeichnet, daß in Formel I $R^{31}$ aus einer, zwei oder drei basischen natürlich vorkommenden Aminosäuren die Reihe Arg, Lys, Hyl, Orn, Cit und/oder His besteht, insbesondere gleich Arg-OH oder Arg-Arg-OH ist.

5. Pharmazeutische Zubereitung nach einem oder mehreren der Ansprüche 1 bis 4, dadurch gekennzeichnet, daß die (A1 bis A21)- und die (B2 bis B29)-Sequenzen in den Insulinderivaten der Formel I die Sequenzen des Human-, Schweine- oder Rinder-Insulins, insbesondere die Sequenzen des Human-Insulins, sind.

6. Pharmazeutische Zubereitung nach einem oder mehreren der Ansprüche 1 bis 5, dadurch gekennzeichnet, daß sie zusätzlich noch mindestens 1 unmodifiziertes Insulin, vorzugsweise Human-Insulin, enthält.

### Patentansprüche für folgende Vertragsstaaten: ES, GR

1. Verfahren zur Herstellung einer pharmazeutischen Zubereitung der Behandlung des Diabetes mellitus, dadurch gekennzeichnet, daß man mindestens ein basisch modifiziertes Insulinderivat mit einem isoelektrischen Punkt zwischen 5,8 und 8,5 der Formel I

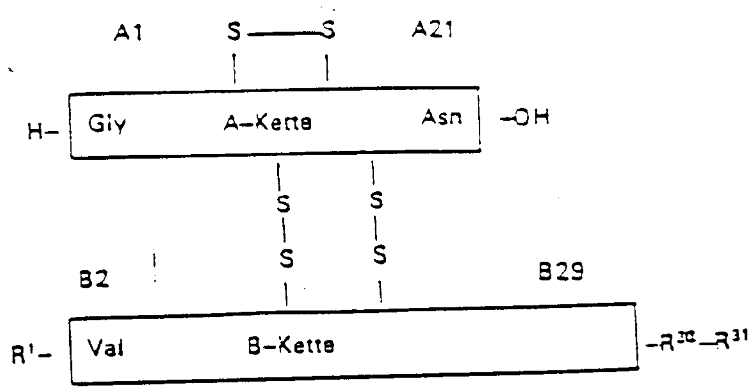

(I)

in welcher $R^1$ H oder H-Phe bedeutet,
$R^{30}$ für den Rest einer neutralen, genetisch kodierbaren L-Aminosäure steht und
$R^{31}$ für eine physiologisch unbedenklich organische Gruppe basischen Charakters mit bis zu 50 C-Atomen steht, an deren Aufbau 0 bis 3 $\alpha$-Aminosäuren beteiligt sind und deren gegebenenfalls vorhandene endständige Carboxylfunktion frei, als Esterfunktion, als Amidfunktion, als Lacton oder $CH_2OH$ reduziert vorliegen kann, und/oder mindestens eines von dessen physiologisch verträglichen Salzen
mit einem pharmazeutisch unbedenklichen Träger sowie ggf. weiteren Zusatz- oder Hilfsstoffen und mit einer Zinkionen liefernden Zinkverbindung derart, daß ein Zinkionengehalt in einem Bereich von oberhalb 1 µg bis etwa 200 µg Zink/IE, vorzugsweise von etwa 1 bis 50 µg/IE, erreicht wird, in eine geeignete pharmazeutische Darreichungsform bringt.

2. Verfahren nach Anspruch 1, dadurch gekennzeichnet, daß man Verbindungen der Formel I mit $R^1$ = H-Phe einsetzt.

3. Verfahren nach Anspruch 1 oder 2, dadurch gekennzeichnet, daß man Verbindungen der Formel I mit $R^{30}$= Ala, Ser oder Thr, vorzugsweise Thr, einsetzt.

4. Verfahren nach einem oder mehreren der Ansprüche 1 bis 3, dadurch gekennzeichnet, daß man Verbindungen der Formel I einsetzt, in welcher $R^{31}$ aus einer, zwei oder drei basischen natürlich vorkommenden Aminosäuren der Reihe Arg, Lys, Hyl, Orn, Cit und/oder His besteht, insbesondere gleich Arg-OH oder Arg-Arg-OH ist.

5. Verfahren nach einem oder mehreren der Ansprüche 1 bis 4, dadurch gekennzeichnet, daß man Verbindungen der Formel I einsetzt, in denen die (A1 bis A21)- und die (B2 bis b 29)-Sequenzen die Sequenzen des Human-, Schweine- oder Rinder-Insulins, insbesondere die Sequenzen des Human-Insulins, sind.

6. Verfahren nach einem oder mehreren der Ansprüche 1 bis 5, dadurch gekennzeichnet, daß man zusätzlich noch mindestens 1 unmodifiziertes Insulin, vorzugsweise Human-Insulin, beifügt.

7. Pharmazeutische Zubereitung, erhalten nach dem Verfahren gemäß einem oder mehreren der Ansprüche 1 bis 6.

## Claims

### Claims for the following Contracting States : AT, BE, CH, DE, FR, GB, IT, LI, LU, NL, SE

1. A pharmaceutical formulation containing at least one insulin derivative modified with a base and having an isoelectric point between 5.8 and 8.5, of the formula I

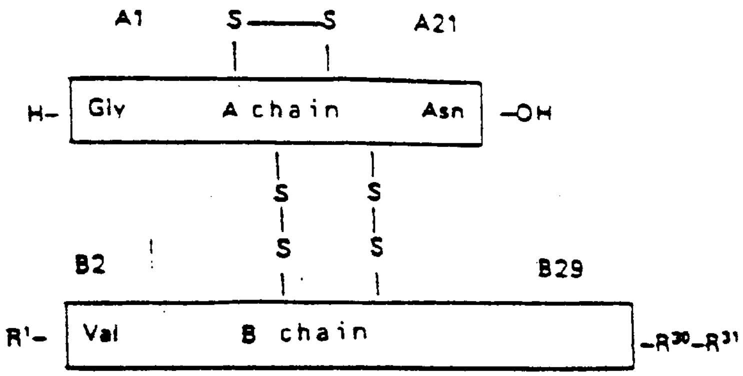

(I)

in which $R^1$ denotes H or H-Phe,
$R^{30}$ represents the radical of a neutral, genetically encodable L-amino acid and
$R^{31}$ represents a physiologically acceptable organic group of basic character having up to 50 carbon atoms, in the build-up of which 0 to 3 $\alpha$-amino acids participate and in which any terminal carboxyl function present can be free, in the ester function form, in the amide function form, in the lactone form or reduced to $CH_2OH$,
and/or at least one of its physiologically tolerated salts in a pharmaceutically acceptable excipient with a content of zinc ions, in which the zinc ion content is in a range from above 1 $\mu$g to about 200 $\mu$g of zinc/IU, preferably from about 1 to 50 $\mu$g/IU.

2. A pharmaceutical formulation as claimed in claim 1, wherein $R^1$ in formula I denotes H-Phe.

3. A pharmaceutical formulation as claimed in claim 1 or 2, wherein $R^{30}$ in formula I denotes Ala, Ser or Thr, preferably Thr.

4. A pharmaceutical formulation as claimed in one or more of claims 1 to 3, wherein $R^{31}$ in formula I is composed of one, two or three basic naturally occurring amino acids of the series comprising Arg, Lys, Hyl, Orn, Cit and/or His, and in particular is Arg-OH or Arg-Arg-OH.

5. A pharmaceutical formulation as claimed in one or more of claims 1 to 4, wherein the (A1 to A21) and the (B2 to B29) sequences in the insulin derivative of formula I are the sequences of human, porcine or bovine insulin, in particular the sequences of human insulin.

6. A pharmaceutical formulation as claimed in one or more of claims 1 to 5, which additionally also contains at least 1 non-modified insulin, preferably human insulin.

**Claims for the following Contracting States : ES, GR**

1. A process for the preparation of a pharmaceutical formulation for the treatment of Diabetes mellitus, which comprises converting into a suitable administration form at least one insulin derivative modified with a base and having an isoelectric point between 5.8 and 8.5, of the formula I

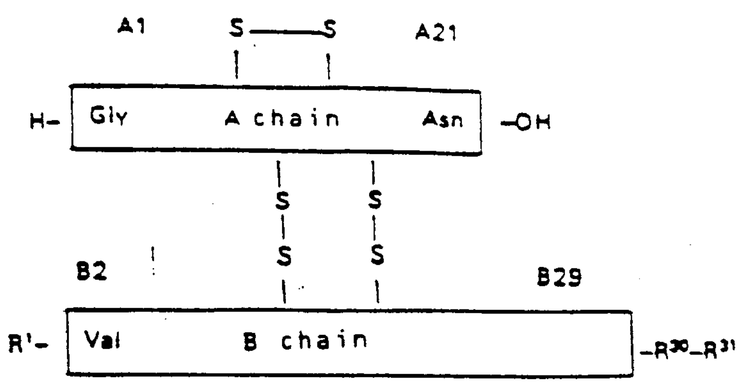

(I)

in which $R^1$ denotes H or H-Phe,
$R^{30}$ represents the radical of a neutral, genetically encodable L-amino acid and
$R^{31}$ represents a physiologically acceptable organic group of basic character having up to 50 carbon atoms, in the build-up of which 0 to 3 $\alpha$-amino acids participate and in which any terminal carboxyl function present can

be free, in the ester function form, in the amide function form, in the lactone form or reduced to $CH_2OH$, and/or at least one of its physiologically tolerated salts with a pharmaceutically acceptable excipient and, where appropriate, further additions or auxiliaries and with a zinc compound donating zinc ions, such that a zinc ion content in a range from above 1 μg to about 200 μg of zinc/IU, preferably from about 1 to 50 μg/IU, is obtained.

2. A process as claimed in claim 1, wherein compounds of the formula I in which $R^1$ denotes H-Phe are used.

3. A process as claimed in claim 1 or 2, wherein compounds of the formula I in which $R^{30}$ denotes Ala, Ser or Thr, preferably Thr are used.

4. A process as clawed in one or more of claims 1 to 3, wherein compounds of the formula I are used in which $R^{31}$ is composed of one, two or three basic naturally occurring amino acids of the series comprising Arg, Lys, Hyl, Orn, Cit and/or His, and in particular is Arg-OH or Arg-Arg-OH.

5. A process as claimed in one or more of claims 1 to 4, wherein compounds of the formula I are used, in which the (A1 to A21) and the (B2 to B29) sequences are the sequences of human, porcine or bovine insulin, in particular the sequences of human insulin.

6. A process as claimed in one or more of claims 1 to 5, wherein additionally also at least 1 non-modified insulin, preferably human insulin, is added.

7. A pharmaceutical formulation, obtained by the process as claimed in one or more of claims 1 to 6.

## Revendications

### Revendications pour les Etats contractants suivants : AT, BE, CH, DE, FR, GB, IT, LI, LU, NL, SE

1. Composition pharmaceutique contenant au moins un dérivé d'insuline à modification basique, ayant un point isoélectrique compris entre 5,8 et 8,5, de formule I

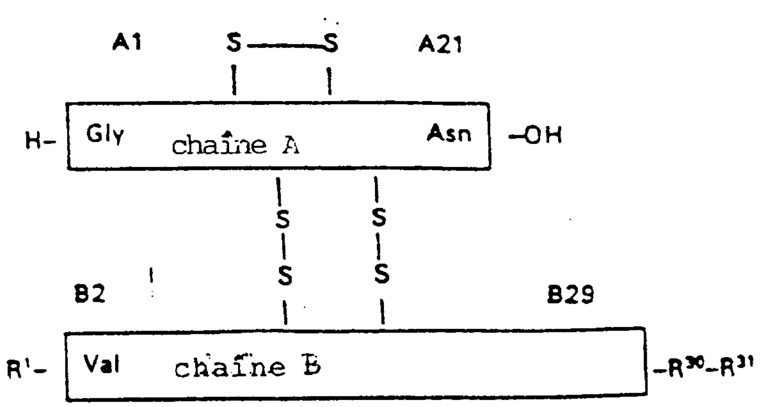


(I)

dans laquelle $R^1$ représente H ou H-Phe,
$R^{30}$ représente le reste d'un L-aminoacide neutre, génétiquement codable, et
$R^{31}$ représente un groupe organique physiologiquement acceptable, à caractère basique, ayant jusqu'à 50 atomes de carbone, à l'édification duquel participent de 0 à 3 α-aminoacides et dont la fonction carboxy terminale éventuellement présente peut se trouver sous forme libre, sous forme de fonction ester, sous forme de fonction amide, sous forme de lactone ou réduite en $CH_2OH$,
et/ou au moins un de ses sels physiologiquement acceptables, dans un véhicule pharmaceutiquement acceptable, ayant une teneur en ions zinc, caractérisée en ce que la teneur en ions zinc se trouve dans une plage allant de plus de 1 μg environ 200 μg de zinc par UI, de préférence d'environ 1 à 50 μg par UI.

2. Composition pharmaceutique selon la revendication 1, caractérisée en ce que, dans la formule 1, $R^1$ représente H-Phe.

3. Composition pharmaceutique selon la revendication 1 ou 2, caractérisée en ce que, dans la formule I, $R^{30}$ représente Ala, Ser ou Thr, de préférence Thr.

4. Composition pharmaceutique selon une ou plusieurs des revendications 1 à 3, caractérisée en ce que, dans la formule I, $R^{31}$ est constitué de 1, 2 ou 3 aminoacides basiques existant dans la nature, choisis parmi Arg, Lys, Hyl, Orn, Cit et/ou His, en particulier est Arg-OH ou Arg-Arg-OH.

5. Composition pharmaceutique selon une ou plusieurs des revendications 1 à 4, caractérisée en ce que

les séquences (A1 à A21) et les séquences (B2 à B29), dans les dérivés d'insuline de formule I, sont les séquences de l'insuline humaine, porcine ou bovine, en particulier les séquences de l'insuline humaine.

6. Composition pharmaceutique selon une ou plusieurs des revendications 1 à 5, caractérisée en ce qu'elle contient en outre au moins une insuline non modifiée, de préférence de l'insuline humaine.

**Claims for the following Contracting States : ES, GR**

1. Procédé pour la préparation d'une composition pharmaceutique pour le traitement du diabète sucré, caractérisée en ce que l'on met sous une forme pharmaceutique appropriée au moins un dérivé d'insuline à modification basique, ayant un point isoélectrique compris 5,8 et 8,5, de formule I

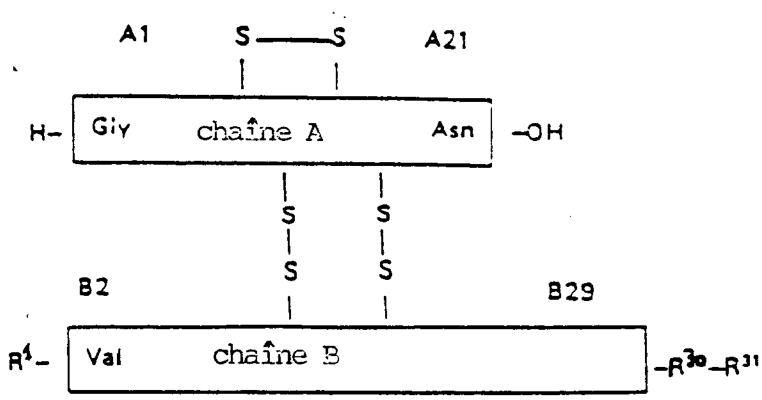

(I)

dans laquelle $R^1$ représente H ou H-Phe,
$R^{30}$ représente le reste d'un L-aminoacide neutre, génétiquement codable, et
$R^{31}$ représente un groupe organique physiologiquement acceptable, à caractère basique, ayant jusqu'à 50 atomes de carbone, à l'édification duquel participent de 0 à 3 $\alpha$-aminoacides, et dont la fonction carboxy terminale éventuellement présente peut se trouver sous forme libre, sous forme de fonction ester, sous forme de fonction amide, sous forme de lactone ou réduite en $CH_2OH$,
et/ou au moins un de ses sels physiologiquement acceptables, avec un véhicule pharmaceutiquement acceptable ainsi qu'éventuellement d'autres additifs ou adjuvants, et avec un composé de zinc donnant des ions zinc, de manière à atteindre une teneur en ions zinc dans une plage allant de plus de 1 $\mu$g à environ 200 $\mu$g de zinc par UI, de préférence d'environ 1 à 50 $\mu$g par UI.

2. Procédé selon la revendication 1, caractérisé en ce que l'on utilise des composés de formule I dans lesquels $R^1$= H-Phe.

3. Procédé selon la revendication 1 ou 2, caractérisé en ce que l'on utilise des composés de formule I dans lesquels $R^{30}$= Ala, Ser ou Thr, de préférence Thr.

4. Procédé selon une ou plusieurs des revendications 1 à 3, caractérisé en ce que l'on utilise des composés de formule I dans lesquels $R^{31}$ est constitué de 1, 2 ou 3 aminoacides basiques existant dans la nature, choisis parmi Arg, Lys, Hyl, Orn, Cit et/ou His, en particulier est Arg-OH ou Arg-Arg-OH.

5. Procédé selon une ou plusieurs des revendications 1 à 4, caractérisé en ce que l'on utilise des composés de formule I dans lesquels les séquences (A1 à A21) et les séquences (B2 à B29) sont les séquences de l'insuline humaine, porcine ou bovine, en particulier les séquences de l'insuline humaine.

6. Procédé selon une ou plusieurs des revendications 1 à 5, caractérisé en ce que l'on ajoute en outre au moins 1 insuline non modifiée, de préférence de l'insuline humaine.

7. Composition pharmaceutique obtenue par le procédé selon une ou plusieurs des revendications 1 à 6.